# EUROPÄISCHE PATENTSCHRIFT

(11) **EP 1 231 920 B1**
(45) Veröffentlichungstag und Bekanntmachung des Hinweises auf die Patenterteilung: **07.02.2007**
(21) Anmeldenummer: 00972763.7
(22) Anmeldetag: 14.10.2000
(51) Int. Cl.: A61K 31/55, A61K 9/08, A61P 11/02, A61P 27/14, A61P 27/02

(54) **VERWENDUNG VON EPINASTIN ZUR BEHANDLUNG VON ALLERGISCHER RHINITIS/KONJUNKTIVITIS**
USE OF EPINASTIN FOR THE TREATMENT OF ALLERGIC RHINITIS/CONJUNCTIVITIS
UTILISATION DE L'EPINASTINE POUR LE TRAITMENT DE LA RHINITE/CONJONCTIVITE ALLERGIQUE

(30) Priorität: 12.11.1999 DE 19954516
(43) Veröffentlichungstag der Anmeldung: 21.08.2002
(73) Patentinhaber: Boehringer Ingelheim International GmbH, 55216 Ingelheim (DE)
(72) Erfinder: TRACH, Volker, 88400 Biberach an der Riss (DE); DUSCHLER, Gerold, 89584 Ehingen (DE)
(86) Internationale Anmeldenummer: PCT/EP2000/010122
(87) Internationale Veröffentlichungsnummer: WO 2001/035962

(56) Entgegenhaltungen:
- EP-A- 0 903 151
- WO-A-97/17971

## Beschreibung

Die Erfindung betrifft die Verwendung topisch applizierbarer wässriger Lösungen enthaltend Epinastin, gegebenenfalls in Form seines Racemats, seiner Enantiomere, sowie gegebenenfalls in Form seiner pharmakologisch unbedenklichen Säureadditionssalze.

### Hintergrund der Erfindung

Unter allergischen Reaktionen des Auges (im folgenden okulare allergische Reaktionen) sind eine Reihe unterschiedlich definierter Krankheitsbilder zu verstehen. Als okulare allergische Reaktionen seien beispielsweise genannt: saisonale allergische Konjunktivitis, perenniale allergische Konjunktivitis, Riesenzellen-Konjunktivitis, vemale Keratokonjunktivitis oder auch atopische Keratokonjunktivitis. Als Beispiele für allergische Reaktionen der Nase (im folgenden nasale allergische Reaktionen) seien beispielsweise die saisonale allergische Rhinitis sowie die perenniale allergische Rhinitis erwähnt.

Der immunologische Mechanismus, der okularen und nasalen allergischen Reaktionen zugrundeliegt umfaßt u.a. Histamin-bedingte Entzündungsprozesse. Die durch die Freisetzung von Histamin bedingten allergischen Reaktionen treten bereits im Frühstadium der eingangs genannten okularen und nasalen allergischen Reaktionen auf.
Ferner können okularen und nasalen allergischen Reaktionen ursächlich die Freisetzung weiterer Mastzellen-Mediatoren sowie toxischer eosinophiler Granula-Proteine und Enzyme zugrunde liegen. Der Zustrom von Neutrophilen und Eosinophilen in das Gewebe der Bindehaut des Auges sowie der Nasenschleimhaut führt dabei zu einer späteren Reaktion (Late-Phase-Reaction, im folgenden LPR). LPR tritt üblicherweise in einem Zeitraum von 3-6 Stunden nach der anfänglichen Histamin-vermittelten allergischen Reaktion auf. LPR ist unter anderem durch das Auftreten von Vasodilatation und Chemosis sowie durch Anschwellen der Conjunctiva (Bindehaut des Auges) sowie der Nasenschleimhaut gekennzeichnet.

Während durch Applikation von Antihistaminika den Histamin-bedingten allergischen Reaktionen entgegengewirkt werden kann, bleibt der Zustrom von Neutrophilen und Eosinophilen in das Gewebe der Bindehaut des Auges sowie der Nasenschleimhaut durch Gabe von reinen Antihistaminika unbeeinflußt.

### Aufgabe der Erfindung

Es ist daher Aufgabe der vorliegenden Erfindung, die Verwendung topisch applizierbarer Lösungen bereitzustellen, die den Zustrom von Neutrophilen und Eosinophilen in das Gewebe der Bindehaut des Auges sowie der Nasenschleimhaut hemmen, das Auftreten von LPR bei allergischer Rhinitis und Konjunktivitis dadurch vermindern oder verhindern und dementsprechend durch eine länger anhaltende Wirkdauer gekennzeichnet sind.

### Detailliertere Beschreibung der Erfindung

Überraschenderweise wurde gefunden, daß topisch applizierbare wässrige Lösungen enthaltend Epinastin, gegebenenfalls in Form seines Racemats, seiner Enantiomere, sowie gegebenenfalls in Form seiner pharmakologisch unbedenklichen Säureadditionssalze, zur Lösung der der Erfindung zugrunde liegenden Aufgabe Verwendung finden können, da sie den Zustrom von Neutrophilen und Eosinophilen in das Gewebe der Bindehaut des Auges sowie der Nasenschleimhaut hemmen, das Auftreten von LPR bei allergischer Rhinitis und Konjunktivitis dadurch vermindern oder verhindern und dementsprechend durch eine länger anhaltende Wirkdauer gekennzeichnet sind.

Die Verbindung Epinastin (3-Amino-9,13b-dihydro-1H-dibenz-[c,f]imidazol[1,5-a]-azepin) sowie dessen Säureadditionssalze sind erstmals in der deutschen Patentanmeldung P 30 08 944.2 beschrieben.

Die Wirkung der topische applizierbaren Epinastin-haltigen Lösungen als Inhibitoren des Eosinophilen und Neutrophilen Zustroms wurde anhand des sogenannten "Passive-Ocular-Anaphylaxis"-Modells in Ratten demonstriert.

### Versuchsbeschreibung:

72 Stunden nach Sensibilisierung der Ratten durch Injektion von Antiserum in die Augenlieder der Testtiere wurde in diesen durch i.v.-Gabe von Ovalbumin eine erneute Provokation induziert. Ein Teil der Versuchstiere wurde 15 Minuten vor der Ovalbumingabe durch Applikation von erfindungsgemäßer Epinastin-haltiger Lösung in den Bindehautsack vorbehandelt. Zwei Stunden nach der Ovalbumingabe wurden die Versuchstiere getötet und die Bindehaut auf den Gehalt an Eosinophilen und Neutrophilen untersucht sowie die Mastzellengranulation bestimmt.

### Ergebnis:

Die mit erfindungsgemäßer Epinastin-Lösung (0,05-0,5%) vorbehandelten Tiere wiesen in der Bindehaut einen deutlich geringeren Gehalt an Eosinophilen auf.
Die mit erfindungsgemäßer Epinastin-Lösung vorbehandelten Tiere wiesen in der Bindehaut einen deutlich geringeren Gehalt an Lymphocyten auf (p<0.01).

In den mit erfindungsgemäßer Epinastin-Lösung vorbehandelten Tieren wurde eine etwa 35%-ige Inhibition der Mastzellen Degranulation bestimmt (p<0.01).

Die Erfindung betrifft folglich die Verwendung topisch applizierbarer wässriger Lösungen enthaltend Epinastin, gegebenenfalls in Form seines Racemats, seiner Enantiomere, sowie gegebenenfalls in Form seiner pharmakologisch unbedenklichen Säureadditionssalze, in einer Konzentration von 0,005 bis 0,5, bevorzugt 0,02 bis 0,1 besonders bevorzugt 0,03 bis 0,07 mg/ml Lösung.

Erfindungsgemäß bevorzugt ist die Verwendung vorstehend genannter topisch applizierbarer wässriger Lösungen enthaltend Epinastin-Hydrochlorid.

Geeignete wässrige Lösemittel sind physiologisch verträgliche wässrige Lösemittel, besonders bevorzugt sind physiologisch verträgliche Kochsalzlösungen.

Erfindungsgemäß werden vorzugsweise topisch applizierbare Lösungen hergestellt, die typischerweise 0,005 bis 0,5, bevorzugt 0,02 bis 0,1 besonders bevorzugt 0,03 bis 0,07 mg/ml Epinastin, gegebenenfalls in Form seines Racemats, seiner Enantiomere, sowie gegebenenfalls in Form seiner pharmakologisch unbedenklichen Säureadditionssalze, sowie physiologische Kochsalzlösungen als Hauptträger enthalten. Der pH-Wert der erfindungsgemäßen Lösungen sollte mit einem geeigneten Puffersystem vorzugsweise im Bereich von 6,5 - 7,2 gehalten werden. Die Präparate können ferner herkömmliche, pharmazeutisch verträgliche Hilfsstoffe, Konservierungsmittel, Stabilisatoren und/oder Penetrationsverstärker enthalten.

Der bevorzugte Träger, der in den erfindungsgemäßen Lösungen verwendet werden kann, ist gereinigtes Wasser und vorzugsweise eine physiologische Kochsalzlösung.

Zu den erfindungsgemäß einsetzbaren Hilfsstoffen gehören, ohne den Gegenstand der Erfindung auf selbige zu beschränken, Viskositätsmittel wie Polyvinylalkohol, Povidone, Hydroxypropylmethylcellulose, Poloxamere, Carboxymethylcellulose, Carbomer und Hydroxyethylcellulose.

Zu bevorzugten Konservierungsmitteln, die in den erfindungsgemäßen Lösungen verwendet werden können, gehören, ohne den Gegenstand der Erfindung auf selbige zu beschränken, Benzalkoniumchlorid, Chlorbutanol, Thimerosal, Phenylquecksilberacetat und Phenylquecksilbemitrat.

Bei den Penetrationsverstärkern kann es sich beispielsweise um oberflächenaktive Mittel, um bestimmte organische Lösemittel wie Dimethylsulfoxid und andere Sulfoxide, Dimethylacetamid und Pyrrolidon, um bestimmte Amide von heterocyclischen Aminen, um Glykole wie Propylenglykol, um Propylencarbonat, um Ölsäure, um Alkylamine und Derivate davon, um verschiedene kationische, anionische, nicht-ionogene und amphotere oberflächenaktive Mittel und um dergleichen handeln.

Mittel zur Einstellung der tonischen Beschaffenheit können je nach Bedarf oder Zweckmäßigkeit zugesetzt werden. Hierzu gehören, ohne die Erfindung auf selbige zu beschränken, Salze und insbesondere Natriumchlorid, Kaliumchlorid, Mannit und Glycerin oder andere geeignete, physiologisch verträgliche Mittel zur Einstellung der Tonizität.

Verschiedene Puffer und Mittel zur Einstellung des pH-Werts können verwendet werden, sofern das erhaltene Präparat physiologisch verträglich ist. Zu entsprechenden Puffern gehören Acetatpuffer, Citratpuffer, Phosphatpuffer und Boratpuffer.

In ähnlicher Weise gehören zu physiologisch verträglichen Antioxidationsmitteln zur erfindungsgemäßen Verwendung, ohne die Erfindung auf selbige zu beschränken, Natriummetabisulfit, Natriumthiosulfat, Acetylcystein, butyliertes Hydroxyanisol und butyliertes Hydroxytoluol.

Weitere Trägerkomponenten, die den erfindungsgemäßen Lösungen einverleibt werden können, sind chelatbildende Mittel. Das bevorzugte chelatbildende Mittel ist Dinatriumedetat (Na-EDTA), wenngleich auch andere chelatbildenden Mittel anstelle von oder in Verbindung mit Dinatriumedetat eingesetzt werden können.

Die vorstehend genannten topisch applizierbaren wässrigen Lösungen, können entweder auf die Bindehaut oder auf die Nasenschleimhaut aufgebracht werden. Lösungen zur ophtalmischen Anwendung sind dabei für die vorliegende Erfindung von gleichrangiger Bedeutung wie nasal zu applizierende Lösungen.

Die vorliegende Erfindung zielt auf die Verwendung der vorstehend genannten topisch applizierbaren wässrigen Lösungen zur Hemmung des Zustroms von Neutrophilen und Eosinophilen in das Gewebe der Bindehaut des Auges oder Gewebe der Nasenschleimhaut.

Die vorliegende Erfindung zielt ferner auf die Verwendung von Epinastin, gegebenenfalls in Form seines Racemats, seiner Enantiomere, sowie gegebenenfalls in Form seiner pharmakologisch unbedenklichen Säureadditionssalze, zur Herstellung der erfindungsgemäßen topisch applizierbaren wässrigen Lösungen zur Behandlung von Störungen der Bindehaut des Auges oder der Nasenschleimhaut, in denen die Hemmung des Zustrom von Neutrophilen und Eosinophilen in das Gewebe der Bindehaut des Auges oder der Nasenschleimhaut bei allergischen Reaktionen einen therapeutischen Nutzen beinhaltet.

Bevorzugt ist die vorstehend genannte Verwendung zur Hemmung von LPR, besonders bevorzugt zur Behandlung der einleitend genannten Erkrankungen.

Die in Tabelle 1 aufgeführten Beispiele erläutern die Erfindung ohne sie auf selbige zu beschränken.

**Tabelle 1:**

| | Lösung 1 0.05% [g/100ml] | Lösung 2 0.01% [g/100ml] | Lösung 3 0.05% [g/100ml] | Lösung 4 0.10% [g/100ml] | Lösung 5 0.01% [g/100ml] | Lösung 6 0.05% [g/100ml] | Lösung 7 0.10% [g/100ml] |
|---|---|---|---|---|---|---|---|
| Epinastin-Hydrochlorid | 0.0500 | 0.0100 | 0.0500 | 0.1000 | 0.0100 | 0.0500 | 0.1000 |
| Na-EDTA | 0.0500 | 0.0500 | 0.0500 | 0.0500 | - | - | - |
| Natriumchlorid | 0.5000 | 0.5000 | 0.5000 | 0.5000 | 0.5000 | 0.5000 | 0.5000 |
| Natriumdihydrogenphosphat-Dihydrat | 0.7800 | 0.7800 | 0.7800 | 0.7800 | 0.4100 | 0.4100 | 0.4100 |
| Benzalkoniumchlorid | 0.0101 | 0.0101 | 0.0101 | 0.0101 | 0.0101 | 0.0101 | 0.0101 |
| Natriumhydroxid | 0.0001 | 0.0001 | 0.0001 | 0.0001 | - | - | - |
| Natriumdihydrogenphosphat-Dihydrat | - | - | - | - | 0.6500 | 0.6500 | 0.6500 |
| Hydroxyethylcellulose | - | - | - | - | 0.1000 | 0.1000 | 0.1000 |
| Wasser | 99.4198 | 99.4598 | 99.4198 | 99.3698 | 99.0749 | 99.0349 | 99.9849 |
| | 100.8100 | 100.8100 | 100.8100 | 100.8100 | 100.7550 | 100.7550 | 100.7550 |

## Patentansprüche

1. Verwendung einer Lösung bestehend aus:
a) Epinastin als aktivem Wirkstoff, gegebenenfalls in Form seines Racemats, seiner Enantiomere, sowie gegebenenfalls in Form seiner pharmakologisch unbedenklichen Säureadditionssalze, in einer Konzentration von 0,0005 bis 0,1 Gew.%,
b) Wasser oder eine physiologische Kochsalzlösung als Lösungsmittel,
c) einen Puffer zum Einstellen eines pH-Werts von 6,5 bis 7,2, gegebenenfalls durch Zugabe von Natriumhydroxid,
d) einem Konservierungsmittel,
und optional
e) chelatbildende Mittel,
f) Viskositätsmittel,
g) Penetrationsverstärkern,
h) Antioxidationsmitteln,
i) und/oder physiologisch verträgliche Mittel zum Einstellen der Tonizität der Lösung.
zur Herstellung eines Medikaments zur topischen Applikation auf die Bindehaut oder Nasenschleimhaut zur Behandlung der späteren Reaktion (late phase reaction) bei allergischer Rhinitis oder Konjunktivitis.

2. Verwendung gemäß Anspruch 1, **dadurch gekennzeichnet, dass** der Wirkstoff Epinastin-Hydrochlorid ist.

3. Verwendung gemäß Anspruch 1 oder 2, **dadurch gekennzeichnet, dass** das Viskositätsmittel ausgewählt ist aus der Gruppe Polyvinylalkohol, Povidone, Hydroxypropylmethylcellulose, Poloxamere, Carboxymethylcellulose, Carbomer und Hydroxyethylcellulose.

4. Verwendung gemäß einem oder mehreren der Ansprüche 1 bis 3, **dadurch gekennzeichnet, dass** das Konservierungsmittel ausgewählt ist aus der Gruppe Benzalkoniumchlorid, Chlorbutanol, Thimerosal, Phenylquecksilberacetat und Phenylquecksilbernitrat

5. Verwendung gemäß einem oder mehreren der Ansprüche 1 bis 4, **dadurch gekennzeichnet, dass** das Penetrationsverstärker ausgewählt ist aus der Gruppe Dimethylsulfoxid, Dimethylacetamid, Pyrrolidon, Propylenglykol, Propylencarbonat und Ölsäure.

6. Verwendung gemäß einem oder mehreren der Ansprüche 1 bis 5, **dadurch gekennzeichnet, dass** das Mittel zum Einstellen der Tonizität ausgewählt ist aus der Gruppe Natriumchlorid, Kaliumchlorid, Mannit und Glycerin.

7. Verwendung gemäß einem oder mehreren der Ansprüche 1 bis 6, **dadurch gekennzeichnet, dass** der Puffer ausgewählt ist aus der Gruppe Acetatpuffer, Citratpuffer, Phosphatpuffer und Boratpuffer.

8. Verwendung gemäß einem oder mehreren der Ansprüche 1 bis 7, **dadurch gekennzeichnet, dass** das Antioxidationsmittel ausgewählt ist aus der Gruppe Natriummetabisulfit, Natriumthiosulfat, Acetylcystein, butyliertes Hydroxyanisol und butyliertes Hydroxytoluol

9. Verwendung gemäß einem oder mehreren der Ansprüche 1 bis 8, **dadurch gekennzeichnet, dass** das chelatbildende Mittel Dinatriumedetat ist.

10. Verwendung gemäß einem oder mehreren der Ansprüche 1 bis 9, **dadurch gekennzeichnet, dass** Epinastinhydrochlorid als Wirkstoff in einer Menge von 0,05 bis 0,1 Gew.% verwendet wird.

11. Verwendung gemäß einem oder mehreren der Ansprüche 1 bis 9, **dadurch gekennzeichnet, daß** Epinastinhydrochlorid, als Wirkstoff in einer Menge von 0,005 bis 0,5 mg/ml verwendet wird.

12. Verwendung gemäß einem oder mehreren der Ansprüche 1 bis 10, **dadurch gekennzeichnet, dass** die Lösung aus Wasser als Lösungsmittel, Epinastin-Hydrochlorid, Natriumchlorid, Natriumhydrogenphophat-dihydrat, Benzalkoniumchlorid, Hydroxyethylcellulose und optional Natrium-EDTA und Natriumhydroxid besteht.

## Claims

1. Use of a solution consisting of:
a) epinastine as an active substance, optionally in the form of its racemate, its enantiomers, and optionally in the form of the pharmacologically acceptable acid addition salts thereof, in a concentration of 0.0005 to 0.1 wt.%,
b) water or a physiological saline solution as solvent,
c) a buffer for adjusting the pH to a value from 6.5 to 7.2, optionally by the addition of sodium hydroxide,
d) a preservative,
and optionally
e) chelating agents,
f) viscosity agents,
g) penetration promoters,
h) antioxidants,
i) and/or physiologically acceptable agents for adjusting the tonicity of the solution,
for preparing a medicament for topical application to the conjunctiva or nasal mucosa for treating late phase reaction in allergic rhinitis or conjunctivitis.

2. Use according to claim 1, **characterised in that** the active substance is epinastine hydrochloride.

3. Use according to claim 1 or 2, **characterised in that** the viscosity agent is selected from among polyvinyl alcohol, povidone, hydroxypropylmethyl cellulose, poloxamers, carboxymethylcellulose, carbomer and hydroxyethyl cellulose.

4. Use according to one or more of claims 1 to 3, **characterised in that** the preservative is selected from among benalkonium chloride, chlorobutanol, thimerosal, phenyl mercury acetate and phenyl mercury nitrate.

5. Use according to one or more of claims 1 to 4, **characterised in that** the penetration promoter is selected from among dimethylsulphoxide, dimethylacetamide, pyrrolidone, propyleneglycol, propylene carbonate and oleic acid.

6. Use according to one or more of claims 1 to 5, **characterised in that** the agent for adjusting tonicity is selected from among sodium chloride, potassium chloride, mannitol and glycerol.

7. Use according to one or more of claims 1 to 6, **characterised in that** the buffer is selected from among acetate buffer, citrate buffer, phosphate buffer and borate buffer.

8. Use according to one or more of claims 1 to 7, **characterised in that** the antioxidant is selected from among sodium metabisulphite, sodium thiosulphate, acetylcysteine, butylated hydroxyanisole and butylated hydroxytoluene.

9. Use according to one or more of claims 1 to 8, **characterised in that** the chelating agent is disodium edentate.

10. Use according to one or more of claims 1 to 9, **characterised in that** the epinastine hydrochloride is used as active substance in an amount of from 0.05 to 0.1 wt.%.

11. Use according to one or more of claims 1 to 9, **characterised in that** the epinastine hydrochloride is used as active substance in an amount of from 0.005 to 0.5 mg/ml.

12. Use according to one or more of claims 1 to 11, **characterised in that** the solution consists of water as solvent, epinastine hydrochloride, sodium chloride, sodium hydrogen phosphate dehydrate, benzalkonium chloride, hydroxyethylcellulose and optionally sodium-EDTA and sodium hydroxide.

## Revendications

1. Utilisastion d'une solution consistant en:
a) de l'epinastine comme principe actif actif, éventuellement sous forme de son racémate, de ses énantiomères, ainsi éventuellement que sous forme de ses sels d'addition d'acide pharmacologiquement acceptables, en une concentration de 0.0005 en 0.1 % en masse,
b) de l'eau ou une solution physiologique de chlorure de sodium comme solvant,
c) un tampon pour l'ajustement d'un pH 6.5 à 7.2, éventuellement par addition d'hydroxyde de sodium,
d) un conservateur,
et éventuellement
e) des agents chélatants,
f) des agents de viscosité,
g) des renforcateurs de pénétration,
h) des antioxydants,
i) et/ou des agents physiologiquement acceptables pour l'ajustement de la tonicité de la solution,
pour la production d'un medicament pour l'application topique sur la conjonctive ou la muqueuse nasale pour le traitement de la réaction ultérieure (late phase reaction) dans le cas de la rhinite allergique ou de la conjonctivite.

2. Utilisation selon la revendication 1 **caractérisée en ce que** le principe actif est le chlorhydrate d'epinastine.

3. Utilisation selon la revendication 1 ou 2 **caractérisée en ce que** l'agent de viscosité est choisi dans le groupe poly(alcool vinylique), Povidone, hydroxypropylméthylcellulose, poloxamères, carboxyméthylcellulose, carbomère et hydroxyéthylcellulose.

4. Utilisation selon une ou plusieurs des revendications 1 à 3 **caractérisée en ce que** le conservateur est choisi dans le groupe chlorure de benzalkonium, chlorobutanol, thimérosal, acétate de phénylmercure et nitrate de phénylmercure.

5. Utilisation selon une ou plusieurs des revendications 1 à 4 **caractérisée en ce que** le renforcateur de pénétration est choisi dans le groupe diméthylsulfoxyde, diméthylacétamide, pyrrolidone, propylèneglycol, carbonate de propylène et acide oléique.

6. Utilisation selon une ou plusieurs des revendications 1 à 5 **caractérisée en ce que** l'agent pour l'ajustement de la tonicité est choisi dans le groupe chlorure de sodium, chlorure de potassium, mannitol et glycérine.

7. Utilisation selon une ou plusieurs des revendications 1 à 6 **caractérisée en ce que** le tampon est choisi dans le groupe tampon acétate, tampon citrate, tampon phosphate et tampon borate.

8. Utilisation selon une ou plusieurs des revendications 1 à 7 **caractérisée en ce que** l'antioxydant est choisi dans le groupe métabisulfite de sodium, thiosulfate de sodium, acétylcystéine, hydroxyamisole butylé et hydroxytoluène butylé.

9. Utilisation selon une ou plusieurs des revendications 1 à 8 **caractérisée en ce que** l'agent chélatant est l'édétate de disodium.

10. Utilisation selon une ou plusieurs des revendications 1 à 9 **caractérisée en ce que** le chlorhydrate d'epinastine est utilisé comme principe actif en une quantité de 0.05 à 0.1 % en masse.

11. Utilisation selon une ou plusieurs des revendications 1 à 9 **caractérisée en ce que** le chlorhydrate d'epinastine est utilisé comme principe actif en une quantité de 0.005 à 0.5 mg/ml.

12. Utilisation selon une ou plusieurs des revendications 1 à 11 **caractérisée en ce que** la solution consiste en eau comme solvant chlorhydrate d'epinastine, chlorure de sodium, hydrogénophosphate de sodium dihydraté, chlorure de benzalkonium, hydroxyéthylcellulose est éventuellement EDTA de sodium et hydroxyde de sodium.
